# EUROPEAN PATENT SPECIFICATION

(11) **EP 0 329 413 B1**
(45) Date of publication and mention of the grant of the patent: **04.08.1993**
(21) Application number: 89301440.7
(22) Date of filing: 15.02.1989
(51) Int. Cl.: C07K 13/00, C12N 15/00

(54) **Polypeptides with cell-spreading activity**
Polypeptide mit Zellausdehnungs-Aktivität
Polypeptides favorisant l'expansion cellulaire

(30) Priority: 16.02.1988 JP 31820/88
(43) Date of publication of application: 23.08.1989
(73) Proprietor: TAKARA SHUZO CO. LTD., Fushimi-ku Kyoto 612 (JP)
(72) Inventor: Kimizuka, Fusao, Ohmihachiman-shi Shiga-ken (JP); Kinoshita, Tatsuru, Kyoto-shi Kyoto-fu (JP); Ohdate, Yoh'ichi, Amagasaki-shi Hyogo-ken (JP); Taguchi, Yuki, Shiga-ken (JP); Kato, Ikunoshin, Uji-shi Kyoto-fu (JP)
(74) Representative: Geering, Keith Edwin

(56) References cited:
- EP-A- 0 207 751
- JOURNAL OF BIOLOGICAL CHEMISTRY, vol. 258, no. 5, 30th March 1983, pages 3332-3340, Washington, D.C., US; M. HAYASHI et al.: "Domain structure of the carboxyl-terminal half of human plasma fibronectin"
- JOURNAL OF BIOLOGICAL CHEMISTRY, vol. 260, no. 24, 26th October 1986, pages 13256-13260, US; S.K. AKIYAMA et al.: "The interaction of fibronectin fragments with fibroblastic cells"

## Description

This invention relates to a protein which has cell-spreading activity like that of fibronectin. More particularly, the invention relates to a polypeptide which has the cell-spreading activity of fibronectin of human origin; and also to a method for the preparation of said polypeptide.

Fibronectin is a multifunctional glycoprotein which is widely distributed in a variety of animal tissues and body fluids and also on the surface of cultured cells and elsewhere. This compound has various physiological effects, such as causing attachment, spreading, migration differentiation, proliferation, and phagocytosis by cells, and participates in such activities as tissue reconstruction, tissue construction, and protection from infection.

Fibronectin is a polypeptide with a molecular weight of about 250,000 and is a dimer with an S-S bond in the vicinity of the C-terminus. The amino acid sequence of this molecule contains 3 different types of internal repeat, and can be classified as types I, II and III. In addition, there are domain structures which have various functions, with the effect of cell attachment and spreading and the ability to bind to collagen, heparin, fibrin, etc. Of these domains, industrial applications of the biological activity related to the cell attachment and spreading domain have been considered; for example, in the preparation of a coating agent for a culture substrate, it is possible to use this function in the preparation of a substrate to which cells will bind. Also, this function can be used as an accelerator of cell binding in such preparations as collyrium, lotions, and agents for the healing of wounds. Cell spreading is a phenomenon that follows after cell attachment. For cells to proliferate, with some exceptions, it is necessary for the phenomenon of spreading to take place, not cell attachment alone.

The basic structure which is the minimum essential structure for the cell-attachment domain of fibronectin is the sequence Arg-Gly-Asp-Ser (Nature, 309 1984 , 30-33). Japanese Laid-Open Patent (Tokuhyo) 84-501548 discloses a peptide with cell-attachment activity, that is a polypeptide of a molecular weight of 11,500 and that contains this sequence among its sequence of 108 amino acid residues.

However, the cell-attachment activity of this polypeptide with a molecular weight of 11,500 is much weaker than that of fibronectin of natural origin, and it is not necessarily possible to make use of it in the practical applications mentioned above. This difficulty is discussed, for example, in J. Biol. Chem., 260 (1985), 13256-13260. Also, we have constructed this polypeptide of 11,500 molecular weight (MW) by genetic engineering, and compared its cell-spreading activity to that of fibronectin of natural origin with the use of normal rat kidney (NRK) cells. The results were that, whereas fibronectin gave noticeable activity at a dose of 0.1-1 µg/well, a dose of 50 µg/well of the 11,500 MW polypeptide did not have any such activity.

This invention identifies the amino acid sequence that has substantial cell-spreading activity as the peptide of the cell-spreading domain of fibronectin and provides a method for producing the same.

The present invention provides polypeptides with cell-spreading activity, which have an amino acid sequence represented by the following general formula [I]: 
X-Glu Gln His Glu Ser Thr Pro Leu Arg Gly
Arg Gln Lys Thr Gly Leu Asp Ser Pro Thr
Gly Ile Asp Phe Ser Asp Ile Thr Ala Asn
Ser Phe Thr Val His Trp Ile Ala Pro Arg
Ala Thr Ile Thr Gly Tyr Arg Ile Arg His
His Pro Glu His Phe Ser Gly Arg Pro Arg
Glu Asp Arg Val Pro His Ser Arg Asn Ser
Ile Thr Leu Thr Asn Leu Thr Pro Gly Thr
Glu Tyr Val Val Ser Ile Val Ala Leu Asn
Gly Arg Glu Glu Ser Pro Leu Leu Ile Gly
Gln Gln Ser Thr Val Ser Asp Val Pro Arg
Asp Leu Glu Val Val Ala Ala Thr Pro Thr
Ser Leu Leu Ile Ser Trp Asp Ala Pro Ala
Val Thr Val Arg Tyr Tyr Arg Ile Thr Tyr
Gly Glu Thr Gly Gly Asn Ser Pro Val Gln
Glu Phe Thr Val Pro Gly Ser Lys Ser Thr
Ala Thr Ile Ser Gly Leu Lys Pro Gly Val
Asp Tyr Thr Ile Thr Val Tyr Ala Val Thr
Gly Arg Gly Asp Ser Pro Ala Ser Ser Lys
Pro Ile Ser Ile Asn Tyr Arg Thr Glu Ile
Asp Lys Pro Ser Gln Met-Y
wherein X is a sequence having the following formula (II) : 
Val Pro Pro Pro Thr Asp Leu Arg Phe Thr
Asn Ile Gly Pro Asp Thr Met Arg Val Thr
Trp Ala Pro Pro Pro Ser Ile Asp Leu Thr
Asn Phe Leu Val Arg Tyr Ser Pro Val Lys
Asn Glu Glu Asp Val Ala Glu Leu Ser Ile
Ser Pro Ser Asp Asn Ala Val Val Leu Thr
Asn Leu Leu Pro Gly Thr Glu Tyr Val Val
Ser Val Ser Ser Val Tyr
or said sequence (II) with certain amino acid(s) or peptide(s) deleted from the N-terminus, and Y is either cysteine or its deletion.

This invention also provides recombinant plasmids which contain DNA which codes for polypeptides (I), and transformants which carry these recombinant plasmids. The present invention further provides a method for the preparation of polypeptides (I) by the cultivation of these transformants and the collection of the polypeptides from the culture medium.

We have found that the polypeptide for which the above-mentioned JP 84-501548 was filed as having cell attachment activity and which is a 11.5-kDa polypeptide (with a sequence of 108 amino acids) of fibronectin of human origin (termed "FN" below), has almost no cell-spreading activity, but that the peptide with the sequence of 283 amino acids (Ala¹²³⁵-Met¹⁵¹⁷) that extends from the N-terminus of said polypeptide does have about the same level of spreading activity as FN; the latter peptide and its preparation by genetic engineering are described in Japanese Patent Application 148/88 of January 5, 1988 and corresponding US Patent Application Serial No.(USSN) 291,894 of December 29, 1988; a copy of the specification of USSN 291,894 as filed is included in the papers initially filed with the present application, and attention is directed to this US document (e.g. pages 3 to 7 and Examples 1,2 and 4) for background information.

In the present specification, superscript numerals affixed to the symbols for amino acids show the number of the amino acid residue counted from the N-terminus of the amino acids of FN based on the EMBL Data Bank.

We have conducted further research work and have prepared by the use of the techniques of genetic engineering polypeptides with cell-spreading activity which have different chain lengths because of the deletion of amino acid or peptide from the N-terminus of the peptide having a sequence of 283 amino acid residues. We have measured the cell-spreading activity of these polypeptides, and have made clear the relationship between the chain length of the peptide and cell-spreading activity. Further, in the course of such research work it has been found that there occurs a remarkable change in the expression of the peptide depending on the amino acid sequence in the region of the N-terminus. As a result it has become possible to identify the sequence of a peptide which can be expressed in large amounts in addition to its high level of cell-spreading activity. This invention is based upon these findings.

The complete amino acid sequence of fibronectin of human origin and its cDNA sequence were published in The EMBO Journal, 4 (1985), 1755-1759. The steps by which the cDNA corresponding to the amino acid sequence of the cell attachment and spreading domain of fibronectin can be cloned are well known. For example, an RNA fraction containing poly(A) from the liver is prepared, and a cDNA library can be prepared by such methods as the Okayama-Berg method or the Gubler-Hoffmann method. Alternatively, such cDNA libraries are commercially available. Thus it is possible to obtain them from, for example, the Clontech Laboratories, Inc. As a method to obtain the desired cDNA clone from the cDNA library, the DNA which corresponds to the amino acid sequence can be used as a probe with the techniques of colony hybridization or plaque hybridization. The clones which hybridize with the probe are selected, and DNA is extracted from the cell pellet or a phage lytic solution and cleaved with restriction enzymes. Insertions are confirmed by the use of electrophoresis. When necessary, Southern blotting is used to check the insert being hybridized with the probe. In the final step, by the study of the base sequence of the insert by the dideoxy method or the like, the identity of the desired clone can be checked.

When necessary, the vector which carried the cDNA coding for the cell-spreading domain of fibronectin can be amplified in the host cells, purified, and cleaved with restriction enzymes so that the cDNA portion can be removed; electrophoresis can be used to purify the cDNA fragments. These cDNA fragments are joined to expression vectors by inframe ligation using DNA ligase, and by their introduction to host cells it is possible to express the cDNA. Any of the known vectors can be used as expression vectors for Escherichia coli as the host, but for preference, a host-vector system which can have its expression induced and which has strong promoter activity is used. As such vectors, there are, for example, a vector which carries the λPL promoter, a vector which carries the lac promoter, a vector which carries the trp promoter, a vector which carries the pst promoter, a vector which carries both the lac and trp promoters, a vector which carries the lpp promoter, a vector which carries both lpp and lac promoters, and other such known vectors (refer to Yoshiyuki Sakai, "Vector DNA", Kodansha Scientific, 1986).

Also, it is possible to use vectors in which exogenous genes can be inserted downstream from a gene for a signal peptide so that the peptide which has been expressed can be secreted.

When a peptide is expressed by the gene for the desired peptide by the use of an expression vector which can be joined downstream from a gene for a signal peptide or by the use of an expression vector directly, by the joining of the vector a certain length of the amino acid sequence of vector origin is sometimes connected to the N-terminus of the desired peptide. The addition of this sequence causes no essential change in the cell-spreading activity of the polypeptide, and it is possible to use the polypeptide as is. However, it is possible, whenever necessary, to remove this sequence by the techniques of gene engineering. This removal involves what is called site-specific mutagenesis, which is a well-established technique.

Generally, when foreign peptides are expressed in Escherichia coli, they hinder the growth of the host cells, so the cells are grown in the presence of a repressor of the promoter; inactivation of the repressor or the addition of an inducer will induce expression of the foreign peptide. When the foreign peptide does not interfere with the growth of the host cells of Escherichia coli, the use of this technique is unnecessary. The polypeptide expressed in this invention does not inhibit the host cells of Escherichia coli, and there is no need to limit induction of its expression.

The vector carrying the gene for the cell-spreading polypeptide can be used to transform Escherichia coli by the usual methods, and these transformant cells may be cultured under conditions suitable for the expression of the polypeptide, whereby the desired polypeptide can be expressed. For confirmation whether the desired polypeptide is being expressed, immunoblotting can be used. For example, buffer which contains SDS is added to the cells and the whole is heated, by which the total protein is made soluble,and after this polyacrylamide gel electrophoresis is conducted and the electrophoresis pattern is transferred to a nitrocellulose or nylon membrane. The membrane is allowed to incubate first with a monoclonal antibody specific for the cell-spreading domain of fibronectin, and then with an enzyme-labelled second antibody. The enzyme activity of the second antibody so bound gives rise to color in a chromogenic substrate, and thus it is possible to verify the presence of the band for the desired polypeptide.

The cell-spreading polypeptide can be purified from cells of Escherichia coli by, for example, the following method. A cell pellet of harvested cells is suspended in buffer, and ultrasonication is applied to give a soluble fraction and an insoluble fraction. The latter is solubilized in a buffer which contains 7 M urea. The soluble fractions are collected, and placed on a Sepharose 4B column bound with the same antibody as that used for immunoblotting, so that purification is effected by affinity binding. As the eluent, buffer in the vicinity of pH 2 is used. By immunoblotting, the fraction with the desired polypeptide is collected, and it is possible to obtain an almost pure polypeptide judged by electrophoresis. When necessary, purification can be further conducted with FPLC (Pharmacia) or HPLC. The polypeptide obtained in this way can be investigated for the presence or absence of cell-spreading activity in, for example, the following way. A buffer in which a sample has been dissolved is made to adsorb onto wells of a microtiter plate, after which a suspension of normal rat kidney (NRK) cells is added, and the plate incubated for 1 hour at 37°C. Then the morphological changes in the cells are observed in order to determine the presence or absence of cell-spreading activity. By comparison of the minimum dose needed for spreading activity to occur in each well with the activity of fibronectin, it is possible to evaluate the strength of the spreading activity.

As a method for the preparation by genetic engineering techniques of polypeptides of various lengths with deletions in the region of the N-terminus of the peptide with a sequence of 283 amino acids, it is convenient to use the plasmid pTF301 (see Example B below corresponding largely to Example 2 of USSN 291,894), which codes for the peptide with the 283 amino acid sequence that has already been cloned. Thus, first, an appropriate restriction enzyme is used to cleave one site slightly upstream of the initiation codon of pTF301 that codes for the sequence Ala¹²³⁵-Met¹⁵¹⁷ of FN, and then exonuclease is used, by which means it is possible to remove the 5′-end of the sequence. By changes in the reaction conditions, it is possible to obtain a plasmid from which appropriate portions of the 5′-terminus of the coding region have been deleted. Then an appropriate restriction enzyme is used to cleave a site slightly downstream from the termination codon of the coding region of these plasmids, and the DNA which has been cleaved is separated by gel electrophoresis, by which it is possible to obtain fragments of cDNA from which various portions of the 5′-terminal strand have been removed. By the insertion of these cDNA fragments into an appropriate expression vector, it is possible to express peptides of various lengths wherein portions of the N-terminal region of the sequence of Ala¹²³⁵-Met¹⁵¹⁷ (283 amino acid residues) have been deleted.

As the expression vector, any of the well-known vectors can be used. We have obtained satisfactory results with direct expression by the use of the pUC-type vectors in which the distance between the ribosome-binding site and the initiation codon has been made optimum.

Also, by joining with a transcription-termination signal downstream from the termination (stop) codon of pUC vectors, it is possible to improve the expression level.

Selection of the recombinants which express the peptide with cell-spreading activity can be done conveniently with immunoscreening. That is, expression vectors to which the cDNA fragments of different lengths have been joined are inserted into cells of Escherichia coli by the usual methods, and the transformants obtained are raised on nitrocellulose filters, after which they are lysed, and the protein from the cells is fixed on the filters. After the filters are blocked with bovine serum albumin or the like, a monoclonal antibody which recognizes the domain of cell spreading of FN is caused to act. The monoclonal antibody bound to the filter is detected by labelling with a second antibody. In this way, it is possible to select recombinants that express the peptide with the domain for cell spreading.

Next, the recombinants so selected are cultured under conditions suitable for expression, and expression of the peptide with the domain for cell spreading is induced. For verification that expression is taking place, immunoblotting can be used. Thus, the whole-cell protein of the cultured cells is lysed by heat treatment in a buffer containing SDS, and separation is conducted by SDS-polyacrylamide electrophoresis, and the electrophoretic pattern is transferred to a nitrocellulose or nylon membrane. After a monoclonal antibody specific for the cell-spreading domain of FN is incubated with the membrane, an enzymelabelled second antibody is applied, and the enzyme activity of the bound antibody gives rise to color in a chromogenic material, whereby it is possible to confirm that there is a band of the peptide with the cell-spreading domain.

Purification of the peptide with the domain for cell spreading from the recombinants can be done, for example, as follows. The cell pellet is suspended in a buffer, and the soluble fraction and insoluble fraction are separated by ultrasonification. The insoluble fraction is solubilized in a buffer which contains 7 M urea. The soluble fractions are pooled, put on a Sepharose 4B column bound with the antibody used in immunoblotting, and affinity purification is carried out. For elution there is used a buffer in the pH region of 2.3. By collection of the desired fractions by immunoblotting, it is possible to collect the peptide with the domain for cell spreading. When necessary, further purification by FPLC and HPLC can be done.

The peptide with the cell-spreading domain thus obtained may be measured for its cell-spreading activity towards NRK (normal rat kidney) cells. The sample is dissolved in a buffer, and used to coat microtiter plate wells, after which NRK cells are added, and the plate is incubated for a fixed time at 37°C. The spreading of the cells is observed under a microscope, and the minimum dose of sample per well that gives rise to the expression of cell-spreading activity is compared to the dose needed of FN of natural origin. In this way, the strength of the cell-spreading activity can be expressed.

By the series of experiments described above, it has been found that the cell-spreading activity of the cell-spreading domain of FN varies with the length of the peptide chain from the N-terminus. The peptide with a molecular mass of 11.5 kDa with 108 amino acids had no cell-spreading activity. As the length from the N-terminus increased, activity abruptly comes to be expressed. With 138 amino acids, activity was about 1/25th that of FN; with 206 amino acids, activity was about 1/3 that of FN; with 258 amino acids or 279 amino acids, activity was about the same as that of FN. These peptides with cell-spreading activity can be used as they are or bound with other peptides by use of a crosslinking agent. As a method for binding with other peptides, there is, for example, the addition of a cysteine residue at the C-terminus of the peptide, and by the use of a bifunctional crosslinking agent such as 3-(2-pyridyldithio)propionic acid-N-hydroxysuccinimide ester, or SPDP, the cysteine residue can be bound to an amino acid residue of another peptide. It is easy to add a cysteine residue to the C-terminus of a peptide by means of genetic engineering. For example, when the gene that codes for the desired peptide is cloned, the addition of the codon that corresponds to cysteine can be added immediately before the termination codon on the 3'-end.

A peptide of this invention should be suited for high level of expression in addition to its strong cell-spreading activity. In this aspect, we have compared the level of expression of various peptides by use of their patterns on SDS-PAGE, and found that the level of expression changes greatly depending on differences in the amino acid sequence in the region of the N-terminus of the peptide. As a result it has been possible to select peptides which have the sequence (I) above.

Among the polypeptides having amino acid sequences represented by the general formula [I], most preferable is one having the following amino acid sequence (279 amino acids):

Pro Thr Asp Leu Arg Phe Thr Asn Ile Gly
Pro Asp Thr Met Arg Val Thr Trp Ala Pro
Pro Pro Ser Ile Asp Leu Thr Asn Phe Leu
Val Arg Tyr Ser Pro Val Lys Asn Glu Glu
Asp Val Ala Glu Leu Ser Ile Ser Pro Ser
Asp Asn Ala Val Val Leu Thr Asn Leu Leu
Pro Gly Thr Glu Tyr Val Val Ser Val Ser
Ser Val Tyr Glu Gln His Glu Ser Thr Pro
Leu Arg Gly Arg Gln Lys Thr Gly Leu Asp
Ser Pro Thr Gly Ile Asp Phe Ser Asp Ile
Thr Ala Asn Ser Phe Thr Val His Trp Ile
Ala Pro Arg Ala Thr Ile Thr Gly Tyr Arg
Ile Arg His His Pro Glu His Phe Ser Gly
Arg Pro Arg Glu Asp Arg Val Pro His Ser
Arg Asn Ser Ile Thr Leu Thr Asn Leu Thr
Pro Gly Thr Glu Tyr Val Val Ser Ile Val
Ala Leu Asn Gly Arg Glu Glu Ser Pro Leu
Leu Ile Gly Gln Gln Ser Thr Val Ser Asp
Val Pro Arg Asp Leu Glu Val Val Ala Ala
Thr Pro Thr Ser Leu Leu Ile Ser Trp Asp

Ala Pro Ala Val Thr Val Arg Tyr Tyr Arg
Ile Thr Tyr Gly Glu Thr Gly Gly Asn Ser
Pro Val Gln Glu Phe Thr Val Pro Gly Ser
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Gln Met

The invention is illustrated in more detail by the following Examples, but is not to be taken to be limited to them.

### Example A

Cloning of cDNA fragment which codes for Ile¹⁴¹⁰-Met¹⁵¹⁷ (108 amino acid residues) of fibronectin :

### (1-1) Preparation of cDNA fragments:

First, 100 µg of a plasmid, pLF5 (reported in Biochemistry, 25 [1986], 4936-4941),which is of 4.1 kilobases and which contains the cDNA sequence which codes for the cell-spreading domain of fibronectin, was put into 200 µl of a reaction mixture containing a buffer for use with the restriction enzyme BalI and 100 units of BalI, and the mixture was incubated for 2 hours at 37°C. The reaction mixture was then put on an HPLC column of DEAE-4000 (6 x 125 mm; Nucleogen) and eluted with a concentration gradient of KCl in a 30 mM potassium phosphate buffer (pH 6.5) which contains 5 M urea, and precipitated with isopropyl alcohol, which gave 7.5 µg of 0.75 kb fragments. Next, these fragments were treated with 30 units of FokI for 1 hour at 37°C, and by the same purification method, 60 ng of 92-bp fragments and 140 ng of 203-bp fragments were obtained.

### (1-2) Preparation of synthetic DNA linker:

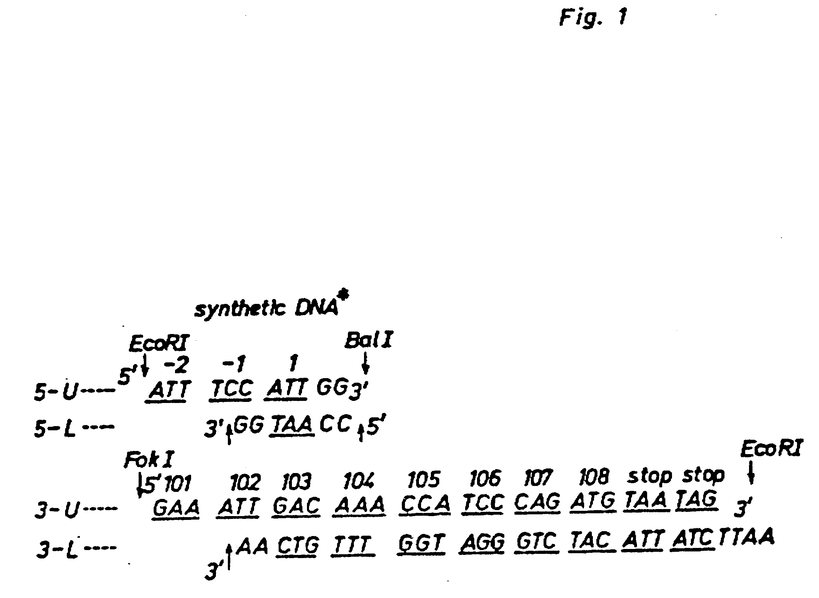

So that the cDNA fragments could be joined to a vector, 5′-end and 3′-end linkers were chemically synthesized by the following method. The base sequence is shown in Fig. 1. The upper strand of the 5′-end linker (chain length, 11), labelled 5 U, the lower strand of the 5′-end linker (chain length, 7), labelled 5 L, the upper strand of the 3′-end liner (chain length, 30), labelled 3 U, and the lower strand of the 3′-end linker (chain length, 30), labelled 3 L, were synthesized by use of the DNA synthesizer of Applied Biosystems, Inc. After deprotection, 5 U and 5 L were purified by ion-exchange HPLC on a TSK gel DEAE-2000 column, and desalted on SepPak (Waters), giving 60 µg and 40 µg, respectively. 3 U and 3 L were separated by electrophoresis using 12% polyacrylamide gel containing 7 M urea, and after removal from the gel, they were desalted in the same way as for the 5 U and 5 L, giving 32 µg and 26 µg, respectively. The base sequences were checked by the solid-phase Maxam-Gilbert method. Each strand was then phosphorylated on its 5′ terminus in the following way. First, 25 µg of DNA was dissolved in 10 µl of distilled water, and put into 20 µl of a solution of buffer for use with polynucleotide inase (PNK) containing 50 mM Tris-HCl, pH 7.6, 10 mM MgCl₂, and 10 mM DTT, 1 mM ATP, and 5 units of PNK; the mixture was incubated for 1 hour at 37°C; then the reaction was stopped by heat treatment at 65°C for 10 minutes. After the phosphorylation, by the annealing of 5 U with 5 L and of 3 U with 3 L, the strands were made double-stranded. That is, equal amounts of 5 U and 5 L were mixed together, and left at 60°C for 10 minutes, after which the mixture was allowed to cool to room temperature gradually, and then cooled further to 15°C. The 3 U and 3 L were treated in the same way.

### (1-3) Binding of cDNA fragments and the linkers:

The two kinds of linker prepared in section (1-2) above and the two kinds of DNA fragment (with 92 bp and with 203 bp) prepared in section (1-1) above were used, and ligated together. For this, 0.5 pmol of each kind of cDNA fragment and 5 pmol of each kind of linker were put into 20 µl of a solution containing a ligation buffer [66 mM Tris-HCl, pH 7.6, and 6.6 mM MgCl₂] and also 0.5 mM APT, 10 mM DTT, and 2.8 units of T4 DNA ligase, at 16°C; the mixture was incubated overnight. The reaction mixture was heated for 10 minutes at 65°C, and then two volumes of cold ethanol was added, and the whole left at -70°C for 30 minutes. After this, the precipitated DNA was collected by centrifugation. This DNA was put into 20 µl of a reaction mixture made of buffer for use with EcoRI and 7.5 units of EcoRI, and incubated for 30 minutes at 37°C. Then the mixture was subjected to polyacrylamide electrophoresis, whereby 336-bp fragments were obtained. These were extracted for 10 hours at 37°C in a gel elution buffer (0.5 M ammonium acetate, 0.01 M magnesium acetate, and 1 mM EDTA), after which the fragments were precipitated with ethanol, and the DNA was collected. This DNA was dissolved in 20 µl of TE (10 mM Tris-HCl and 1 mM EDTA, pH 8.0).

### (1-4) Binding of cDNA fragments bound with linkers to a vector:

First, 1 ug of the secretion-expression vector pIN-III-ompA-l (reported in the EMBO Journal, 3 [1984], 2437-2442) was incubated together with 5 units of EcoRI in buffer for use with EcoRI for 1 hour at 37°C; next, 0.5 unit of alkaline phosphatase was added and incubation continued for another hour. The reaction mixture was treated twice with phenol, and ethanol precipitation was used to give 0.5 µg of DNA. This DNA was cleaved with EcoRI, and then 0.1 µg of dephosphorylated pIN-III-ompA-l vector was put into a mixture with 0.1 µg of the DNA fragments obtained in section (1-3) above together with 10 µl of buffer for use with T4 DNA ligase, 0.5 mM ATP, 10 mM DTT, and 1.8 units of T4 DNA ligase, in a total volume for the reaction mixture of 30 µl. This mixture was incubated overnight at 16°C. Then 15 µl of this reaction mixture was used in the transformation of Escherichia coli cells.

### (1-5) Transformation of cells of Escherichia coli:

Cells of Escherichia coli SB221 (The EMBO EMBO Journal, 3 [1984], 2437-2442) were put into a 20 ml test tube containing 5 ml of L-broth (Bactotrypton, 10 g/l; yeast extract, 5 g/l; NaCl, 5 g/l; pH 7.2) and cultured overnight with agitation at 37°C. Then 0.05 ml of this culture broth was used to inoculate 5 ml of fresh L-broth, and culture was continued with agitation until the absorbance at 600 nm reached 0.6. This culture broth was cooled over ice and centrifuged for 5 minutes at 5000 rpm at 4°C. The supernatant was removed. This was mixed with 2.5 ml of an ice-cold 0.1 M MgCl₂ solution, after which it was centrifuged in the same way and the supernatant removed. This was mixed with 1.25 ml of ice-cold 0.1 M CaCl₂, immediately mixed with ice-water, and left for 30 minutes. The mixture was centrifuged and the supernatant removed; then it was mixed with 0.1 ml of ice-cold 0.1 M CaCl₂ to suspend it rapidly. The suspension was mixed with 10 µl of the reaction mixture obtained in section (1-4) above, and left for 30 minutes in an ice bath. Next, it was heated to 42°C for 2 minutes. Then, 1 ml of L-broth heated to 37°C was added, and the whole was incubated for 30 minutes at 37°C. After this, the mixture was cultured for 1 hour at 37°C with agitation. The culture broth was spread on L agar medium that contained 50 µg/ml ampicillin, and colonies which grew after overnight culture at 37°C were isolated. About 200 colonies were obtained, and fourteen of these were analyzed for plasmids.

### (1-6) Analysis of plasmids:

After 14 transformants were cultured with agitation overnight in 1.5 ml of L-broth containing 50 µg/ml ampicillin, the cultures were centrifuged and the cell pellets were obtained. The cell pellets were suspended in 100 µl of solution A (50 mM glucose, 10 mM EDTA, 25 mM Tris-HCl, pH 8.0, and 2 mg/ml lysozyme), and left at room temperature for 5 minutes. Then 200 µl of solution B (0.2 N NaOH and 1 % SDS) was added to the suspension, and the container was rapidly inverted five times and then left at 0°C for 10 minutes. To the containiner, 150 µl of 5 M potassium acetate (pH 5.0) was added, followed by the addition of two volumes of cold ethanol, and the mixture was left at -70°C for 30 minutes, after which it was centrifuged and the DNA was obtained. The DNA was washed with 80% ethanol, and the ethanol and water were removed under reduced pressure. The residue was dissolved in a small amount of TE. One portion of the solution (0.1 µg) was allowed to react for 1 hour at 37°C in 10 µl of a reaction mixture which contains 5 units of EcoRI in buffer for use with EcoRI. Then the reaction mixture was separated by agarose electrophoresis, and the expected bands for DNA (at 0.33 kb) were checked by ethidium bromide staining. The results showed that nine of the clones gave the desired band. The plasmids extracted from these nine clones were doubly digested with the use of PvuII and HindIII. Then the orientation of the inserted fragments was identified. It was found that eight of the clones had the inserts with the correct orientation. Of these eight clones, the plasmids from five clones were doubly digested with the use of XbaI and HindIII, and the DNA that contained the inserted fragments was removed, and subcloned into the phages M13mp18 and mp19. The base sequences were analyzed by the dideoxy method. Three of the clones were found to contain the desired sequence. The recombinant plasmids obtained in this way were named pTF101.

### (1-7) Purification of plasmids:

The pTF101 obtained as described above in section (1-6) was used to transform Escherichia coli cells (SB221/pTF 101) and such transformed cells were cultured in 5 ml of L-broth overnight with agitation at 37°C. The culture was transferred to 500 ml of the same culture broth and culture was continued until the absorbance at 660 nm reached 0.6, at which time 170 µg/ml chloramphenicol was added, and cultured was continued once more for 10-12 hours. The culture broth was centrifuged for 10 minutes at 5000 rpm, and the cell pellet was washed in STE (10 mM Tris-HCl, pH 8, 100 mM NaCl, and 1 mM EDTA), and centrifuged again in the same way in order to obtain the cell pellet. The pellet was suspended in 10 ml of the solution A, and kept at room temperature for 5 minutes before 20 ml of the solution B was added with rapid stirring, and the suspension was left for 5 minutes at 0°C. To this suspension, 15 ml of 3 M potassium acetate was added with rapid stirring, and the suspension was left at 0°C for 10 minutes. The mixture was centrifuged for 60 minutes at 8000 rpm and the supernatant was obtained, to which 27 ml (0.6 volume) of isopropyl alcohol was added, and the mixture was left at room temperature for 15 minutes. This mixture was centrifuged and the precipitate was collected and washed in 80% ethanol. The ethanol and water in the resulting mixture was removed under reduced pressure and the residue was dried. This residue was dissolved in 4 ml of TE and to this solution 4.75 g of cesium chloride was added, followed by the addition of 5 mg/ml ethidium bromide in the volume of 420 µl; the mixture was left for 30 minutes at 0°C. The mixture was then centrifuged for 10 minutes at 8000 rpm, and the supernatant was put into a Bechman Quick-seal tube and ultracentrifuged at 55000 rpm overnight. Then, under ultraviolet light, plasmid bands were removed by the use of a syringe. The resulting mixture was extracted seven times in equal volumes of isopropyl alcohol and the remaining ethidium bromide was removed, after which ethanol precipitation was done twice, and then the remaining mixture was washed in 80% ethanol, dried under reduced pressures, and dissolved in a drop of TE (yield, 760 µg).

### Example B

Cloning of the cDNA fragment that codes for Ala¹²³⁵-Met¹⁵¹⁷ (283 amino acid residues) of fibronectin :
First, 50 µg of the plasmid pLF5 was put into 200 µl of a reaction mixture containing 200 units of EcoRI methylase in a buffer for use with EcoRI methylase (100 mM Tris-HCl, pH 8.0, 2 mM DTT, 10 mM EDTA, and 80 µM S-adenosylmethionine), and the mixture was incubated for 60 minutes at 37°C to bring about protection of the EcoRI sites. Then the reaction was stopped by heating at 65°C for 20 minutes, and 96 units of PvuII in buffer for use with PvuII was added; 300 µl of the reaction mixture was incubated for 60 minutes at 37°C. This mixture was separated by agarose electrophoresis, and the slice of gel containing 0.60 kb band was cut out. This slice was put into a dialysis tube that contained elution buffer (5 mM Tris-acetate buffer, pH 8.0, and 1 mM EDTA), and eluted by electrophoresis in the same buffer, by which means the DNA was eluted. The elution fluid was extracted twice with phenol, and a 1/10 volume of 3 M sodium acetate was added, followed by the addition of two volumes of ethanol, and ethanol precipitation was conducted twice. Then the resulting mixture was washed in 80% ethanol, dried, and dissolved in a small amount of TE (yield, 1.2 µg).

Also, 140 µg of the plasmid pTF101 obtained in Example A was put into 200 µl of a reaction solution containing 144 units of PvuII, and the mixture was incubated for 60 minutes at 37°C. Then 150 units of EcoRI was added, and the reaction solution brought to 240 µl, and incubated for 60 minutes at 37°C. The result was separated by polyacrylamide electrophoresis, and the bands at 0.25 kb were cut out. The DNA was extracted from these bands of gel as described above, and the DNA was collected by ethanol precipitation (yield, 0.4 µg). Then 1.25 µg of the 0.6-kb fragments obtained here and 0.4 µg of the 0.25-kb fragments were put into a ligation buffer which contained 2.8 units of T4 DNA ligase, 0.5 mM ATP, and 10 mM DTT in 50 µl, and the mixture was incubated for 30 minutes at 16°C. Then 92 pmol of phosphorylated linker (pCCGAATTGG) and 1.4 units of T4 DNA ligase were added to the reaction mixture, which was brought to 70 µl and incubated overnight at 10°C. The reaction was stopped by heating at 65°C for 10 minutes, and the buffer was modified to be suitable for EcoRI; 30 units of EcoRI was added, and reaction was allowed to take place for 60 minutes at 37°C. Then the reaction mixture was separated by agarose electrophoresis, and the 0.86-kb bands were cut out. The DNA in the bands was obtained by the method described above (yield, about 25 ng). Then 20 ng of the 0.86-kb fragments were cleaved with EcoRI, and put into a ligation buffer which contains 0.1 µg of dephosphorylated vector pIN-III-OMPA-l, 2.8 units of T4DNA ligase, 0.5 mM ATP, and 10 mM DTT in a total volume of 20 µl; the mixture was incubated for 10 hours at 16°C and half of the reaction was used to transform Escherichia coli SB 221 cells by the method described above. Of the transformants obtained, 48 of the clones were analysed for plasmids by the methods described above, and it was found that the plasmids included the desired fragments in six of the clones. These six plasmids were digested with BamHI, and the fragments which were produced were analysed. Five of the clones contained the desired fragments inserted in the correct orientation. Three of these five clones were studied for their base sequence. One of the clones was found to have the correct base sequence. This recombinant plasmid was named pTF301, and the cells of Escherichia coli SB221 that carry this plasmid were designated SB221/pTF301.

### Example 1.

### (1) Preparation of DNA fragments.

First, 100 µg of the plasmid pTF301 which codes for peptide 283AA with cell-spreading activity was mixed with a buffer for use with the restriction enzyme XbaI and with 120 units of XbaI in a total volume of 300 µl, and incubated at 37°C for 2 hours. Then, the DNA was collected by ethanol precipitation. Half of the amount obtained was put into 375 µl of a reaction mixture of buffer for use with BAL 31 nuclease which contained 36 units of BAL 31 nuclease-S, and the mixture was incubated at 30°C; from 2 minutes of incubation to 8 minutes, 28 µl of the reaction mixture was sampled every 30 seconds, and each sample was added to 20 µl of 0.5 M EDTA to stop the reaction. The samples were pooled, and DNA was obtained by phenol treatment and then ethanol precipitation. The DNA obtained was put into 200 µl of a reaction mixture of 7 mM Tris-HCl, pH 7.5, which contained 0.1 mM EDTA, 10 mM NaCl, 7 mM MgCl₂, 0.1 mM dATP, dGTP, dCTP, and dTTP, and 2 units of Klenow fragment. The mixture was incubated for 20 minutes at 37°C. The reaction was stopped by treatment at 65°C for 10 minutes, and the reaction mixture was adjusted to the composition of buffer for use with HindIII. Then, to 250 µl of this reaction mixture, 60 units for HindIII was added, and the mixture was incubated for 1 hour at 37°C. This mixture was separated by agarose gel electrophoresis, and the fragments that correspond to the size of 0.5-0.8 kb were cut out; 1 µg was obtained.

### (2) Cloning into pUC119N.

First, 0.65 µg of the DNA fragments obtained in section (1) above was put into 30 µl of a ligase buffer which contained 0.5 µg of phosphorylated NcoI linker (d[pAGCCATGGCT]), 2.8 units of T4 DNA ligase, 0.5 mM ATP, and 10 mM DTT; and the mixture was incubated overnight at 10°C. After the reaction was stopped by being heated at 65°C for 10 minutes, 24 units of NcoI and 12 units of HindIII were added to this reaction mixture and the mixture was incubated in a total volume of 50 µl for 1 hour at 37°C. This was put on a 1-ml column of Sepharose CL-4B; the free linker was eluted with STE buffer (100 mM NaCl, 10 mM Tris-HCl, and 1 mM EDTA, pH 8.0). Then 300 µl of DNA fraction was obtained and concentrated to 55 µl. Of this, 5.5 µl was added to 1 µl of a solution that contained 0.2 µg of the plasmid pUC119 that had been dephosphorylated by treatment with NcoI and HindIII. To this, 65 µl of solution A and 6.5 µl of solution B from a DNA ligation kit (Takara Shuzo Co., Ltd.) were added, and the mixture was incubated overnight at 16°C. Then 20 µl of the reaction mixture was used to transform cells of Escherichia coli HB 101.

In addition, pUC119N was obtained by creation of a NcoI site surrounding the translation initiation codon of the commercially available vector pUC119 (Takara Shuzo Co., Ltd.); the distance between the ribosome-binding site and initiation codon was also changed from 7 to 8 bases.

### (3) Screening of the expression plasmids.

The transformants obtained in section (2) above were transferred to a nitrocellulose filter (BA85; S & S) on L-agar medium containing 50 µg/ml ampicillin, and cultured overnight at 37°C. The colonies that grew were brought into chloroform vapor for 15 minutes, and the nitrocellulose filter was put into a solution of 50 mM Tris-HCl, pH 7.5, containing 150 mM NaCl, 5 mM MgCl₂, 3% bovine serum albumin, 1 µg/ml DNase, and 40 µg/ml lysozyme; the mixture was incubated overnight at room temperature. The filter was treated with an anti-FN monoclonal antibody, FN-10 (Takara Shuzo Co., Ltd.) which specifically recognizes the cell-spreading domain of FN, and a second antibody labelled with perodixase was applied, and the hydrogen peroxide gave rise to color in the presence of 4-chloro-1-naphthol, by which the transformants which had appeared were found. By this first screening, 52 clones were selected from 1300 clones, and each was cultured overnight with agitation at 37°C on 5 ml of L-broth containing 50 µg/ml ampicillin. The whole-cell protein of the cells obtained was separated by SDS-polyacrylamide gel electrophoresis (SDS-PAGE), and immunoblotting was applied, in which the anti-FN monoclonal antibody FN-10 was allowed to react. It was found that polypeptides of molecular masses of 22 kDa-32 kDa had been produced. For 11 clones, the base sequence of the 5′-end of the inserted fragments was identified; they were found to code amino acid sequences which were respectively 279, 258, 219, 213, 207, 206, 198, 195, 190, 186, and 178 long counting from Met¹⁵¹⁷ as the C-terminus.

The amounts of these peptides which were expressed were compared on SDS-PAGE; the peptide which was expressed in the greatest amount was a peptide with a sequence 279 amino acids long (accounting for about 20% of the total whole-cell protein); next were peptides with sequence 206 and 258 amino acids long.

The plasmid which expressed the peptide with the sequence 279 amino acids long was designated pTFD707, and the plasmid which expressed the peptide with the sequence 258 amino acids long was designated pTFD202. At the N-terminus of the peptides expressed by pTFD707 and pTFD202, there was a sequence (GCT) attached that corresponded to alanine of vector origin. This sequence was removed by site-specific mutagenesis (a procedure used,for example, in Example 4 of USSN 291,894). Also, in order to raise the level of expression, the HindIII-SalI fragment with a l pp terminator sequence was removed from the secretion-expression vector pIN-III-ompA-I, and joined with the HindIII-SalI site of pTFD707 and pTFD202, by which means plasmids designated pTF7021 and pTF2021 were obtained.

Escherichia coli JM109 carrying pTF7021 was designated Escherichia coli JM 109/pTF7021, and Escherichia coli coli JM109 carrying pTF2021 was designated Escherichia coli JM109/pTF2021; the original forms were deposited at the Fermentation Research Institute of the Agency for Science and Technology, Japan, as follows: the original form of pTF2021 as FERM BP-1940 and the original form of pTF7021 as FERM BP-1941, both on 3rd February 1988.

When JM109/pTF7021 cells were cultured and the expressed polypeptide with cell-spreading activity was investigated, it was found that this peptide accounted for at least 30% of the peptides expressed in terms of whole-cell protein.

### (4) Cloning and expression of the DNA fragment that code for the Asn¹³⁸⁰-Met¹⁵¹⁷ sequence of 138 amino acids of FN.

First, a 1.3-kb fragment of pTF301 was obtained by restriction degradation of EcoRI and SalI, and purified by gel electrophoresis. Separately, a 2.15-kb fragment of pUC18 was obtained by restriction degradation by EcoRI and SalI and purified in the same way. The two kinds of fragment were joined by the use of T4 DNA ligase, and used to transform Escherichia coli HB101. The transformants obtained were cultured and their expression was checked by the use of immunoblotting.

### Example 2.

### Purification of the peptide with the cell-spreading domain.

Plasmid pTF7021 obtained by the joining of an expression vector and the DNA that codes for the Pro¹²³⁹-Met¹⁵¹⁷ sequence of FN (a sequence of 279 amino acid residues) was introduced into Escherichia coli JM109, giving Escherichia coli JM109/pTF7021, and these cells were cultured overnight with shaking at 37°C in 5 ml of L-broth containing 50 µg/ml ampicillin in a test tube. This was used to inoculate a 2-1 Erlenmeyer flask containing 500 ml of the same medium, and culture was continued at an agitation rate of 180 r.p.m. When absorbance at 660 nm had reached 0.3, 2 mM IPTC (isopropyl-β-thiogalactoside) was added, and the cells were harvested 20 hours later. One portion of the cells obtained was used in immunoblotting. The whole-cell protein was isolated by SDS-PAGE, and the electrophoretic pattern was transferred to a nitrocellulose membrane. Then a monoclonal antibody (FN-10, Takara Shuzo Co., Ltd.) that specifically recognizes the cell-spreading domain of FN was applied, after which a second antibody labelled with peroxidase was applied. The activity of the peroxidase bound to the second antibody gave rise to color in the presence of 4-chloronaphthol and hydrogen peroxide, and it was found that the desired band in the region of 35 kDa was present. Next, the whole-cell pellet was suspended in a solution of 10 mM Tris-HCl, pH 7.5, containing 5 mM EDTA and 5 mM mercaptoethanol, and ultrasonification was conducted. This suspension was centrifuged and the supernatant was obtained and dialyzed against 20 mM Tris-HCl (pH 7.5). The inner dialysis liquid was put on a Sepharose 4B column (8 ml) bound with the monoclonal antibody FN-10. The column was washed with washing buffer A (20 mM Tris-HCl, pH 8.0, and 0.15 M KCl), and then with washing buffer B (20 mM Tris-HCl, pH 6.4, and 0.15 M KCl). Finally, the eluting buffer (50 mM glycine-HCl, pH 2.3, and 0.2 M KCl) was used, and fractions were obtained. The desired fractions were obtained by immunoblotting, desalted, and lyophilized, and about 5 mg of almost pure peptide was obtained by electrophoresis. Next, said peptide was treated with aminopeptidase P (Enzyme Handbook, p. 534 (1983), Asakura Publishers), and the N-terminal methionine was removed, after which the peptide was purified again by the same method as described above. The 10 or so amino acid residues in the amino acid sequence starting from the N-terminus were studied, and it was found that the sequence was Pro-Thr-Asp-Leu-Arg-Phe-Thr-Asn-Ile-Gly, which agreed with the N-terminal sequence of the desired peptide.

The recombinants which correspond respectively to the peptides with sequences 258, 219, 206, 178, and 138 amino acids long were also cultured by the same method and purified; each peptide was isolated, and the structure of the N-terminal region was identified.

### Example 3. Measurement of cell-spreading activity.

The cell-spreading activity of all of the polypeptides obtained in Example 2 and of FN and the peptides with 283 and 108 amino acids was measured by the method of Ruoslahti et al. (Methods in Enzymology, 82, 803-831, 1981). The samples were diluted stepwise in physiological saline and distilled water, and 50 µl aliquots were injected into the wells of a 96-well microtitre plate, which was then incubated overnight at 4°C in order to allow the samples to adhere to the wells. Then, phosphate-buffered saline (PBS) was used to wash the plate twice, 100 µl of 3% BSA was added to each well, and the plate was incubated for one hour at 37°C. The plate was washed twice with PBS, and then normal rat kidney (NRK-49F) cells suspended to a concentration of 10⁶ cells/ml in Eagle's Minimum Essential Medium (MEM) were added in the amount of 100 µl/well, and the plate was incubated for 2-3 hours at 37°C. The NRK-49F cells that were used were obtained as a lyophilized strain for storage, and first preincubated and then treated with trypsin before use. The spreading of the cells was observed under a microscope, and the minimum dose needed to have cell-spreading activity was calculated. These results are shown in Table 1.

Table 1 also shows the amount of peptide expressed by the transformant recombinants, relative to the amount of the peptide with the sequence of 283 amino acids given as +.

**Table 1**

| Polypeptide (length of amino acid sequence) | Minimum dose for cell spreading µg/well (p mole/well) | Relative amount expressed |
|---|---|---|
| Ile¹⁴¹⁰-Met¹⁵¹⁷ ( 108) | >50 (>4400) | |
| Asn¹³⁸⁰-Met¹⁵¹⁷ ( 138) | 0.31 ( 19.7) | |
| Ala¹³⁴⁰-Met¹⁵¹⁷ ( 178) | 0.08 ( 4.0) | |
| Glu¹³¹²-Met¹⁵¹⁷ ( 206) | 0.05 ( 2.2) | +++ |
| Pro¹²⁹⁹-Met¹⁵¹⁷ ( 219) | 0.04 ( 1.8) | ++ |
| Pro¹²⁶⁰-Met¹⁵¹⁷ ( 258) | 0.03 ( 1.2) | +++ |
| Pro¹²³⁹-Met¹⁵¹⁷ ( 279) | 0.03 ( 1.0) | ++++ |
| Ala¹²³⁵-Met¹⁵¹⁷ ( 283) | 0.03 ( 1.0) | + |
| FN (2324) | 0.18 ( 0.8) | |

As explained above in detail, this invention provides a peptide which has cell-spreading activity essentially the same as that of FN, and also provides a method for its preparation by the use of genetic engineering. The polypeptides mentioned above can be used in pharmaneutical preparations for such uses as for the healing of wounds, in collyria, for the prevention of metastases from cancer, for the implantation of artificial organs into the body, and the like. It can also be used in cosmetics, toothpaste, and the like.

## Claims

1. A polypeptide with cell-spreading activity which has an amino acid sequence represented by the general formula : 
X-Glu Gln His Glu Ser Thr Pro Leu Arg Gly
Arg Gln Lys Thr Gly Leu Asp Ser Pro Thr
Gly Ile Asp Phe Ser Asp Ile Thr Ala Asn
Ser Phe Thr Val His Trp Ile Ala Pro Arg
Ala Thr Ile Thr Gly Tyr Arg Ile Arg His
His Pro Glu His Phe Ser Gly Arg Pro Arg
Glu Asp Arg Val Pro His Ser Arg Asn Ser
Ile Thr Leu Thr Asn Leu Thr Pro Gly Thr
Glu Tyr Val Val Ser Ile Val Ala Leu Asn
Gly Arg Glu Glu Ser Pro Leu Leu Ile Gly
Gln Gln Ser Thr Val Ser Asp Val Pro Arg
Asp Leu Glu Val Val Ala Ala Thr Pro Thr
Ser Leu Leu Ile Ser Trp Asp Ala Pro Ala
Val Thr Val Arg Tyr Tyr Arg Ile Thr Tyr
Gly Glu Thr Gly Gly Asn Ser Pro Val Gln
Glu Phe Thr Val Pro Gly Ser Lys Ser Thr
Ala Thr Ile Ser Gly Leu Lys Pro Gly Val
Asp Tyr Thr Ile Thr Val Tyr Ala Val Thr
Gly Arg Gly Asp Ser Pro Ala Ser Ser Lys
Pro Ile Ser Ile Asn Tyr Arg Thr Glu Ile
Asp Lys Pro Ser Gln Met-Y,
 wherein X is a sequence having the following formula (II) : 
Val Pro Pro Pro Thr Asp Leu Arg Phe Thr

Asn Ile Gly Pro Asp Thr Met Arg Val Thr

Trp Ala Pro Pro Pro Ser Ile Asp Leu Thr

Asn Phe Leu Val Arg Tyr Ser Pro Val Lys

Asn Glu Glu Asp Val Ala Glu Leu Ser Ile

Ser Pro Ser Asp Asn Ala Val Val Leu Thr

Asn Leu Leu Pro Gly Thr Glu Tyr Val Val

Ser Val Ser Ser Val Tyr
 or said sequence (II) with certain amino acid(s) or peptide(s) deleted from the N-terminus, and Y is either cysteine or its deletion.

2. A polypeptide with cell-spreading activity according to claim 1 which has the following amino acid sequence:

Pro Thr Asp Leu Arg Phe Thr Asn Ile Gly

Pro Asp Thr Met Arg Val Thr Trp Ala Pro

Pro Pro Ser Ile Asp Leu Thr Asn Phe Leu

Val Arg Tyr Ser Pro Val Lys Asn Glu Glu

Asp Val Ala Glu Leu Ser Ile Ser Pro Ser

Asp Asn Ala Val Val Leu Thr Asn Leu Leu

Pro Gly Thr Glu Tyr Val Val Ser Val Ser

Ser Val Tyr Glu Gln His Glu Ser Thr Pro

Leu Arg Gly Arg Gln Lys Thr Gly Leu Asp
Ser Pro Thr Gly Ile Asp Phe Ser Asp Ile
Thr Ala Asn Ser Phe Thr Val His Trp Ile
Ala Pro Arg Ala Thr Ile Thr Gly Tyr Arg
Ile Arg His His Pro Glu His Phe Ser Gly
Arg Pro Arg Glu Asp Arg Val Pro His Ser
Arg Asn Ser Ile Thr Leu Thr Asn Leu Thr
Pro Gly Thr Glu Tyr Val Val Ser Ile Val
Ala Leu Asn Gly Arg Glu Glu Ser Pro Leu
Leu Ile Gly Gln Gln Ser Thr Val Ser Asp
Val Pro Arg Asp Leu Glu Val Val Ala Ala
Thr Pro Thr Ser Leu Leu Ile Ser Trp Asp
Ala Pro Ala Val Thr Val Arg Tyr Tyr Arg
Ile Thr Tyr Gly Glu Thr Gly Gly Asn Ser
Pro Val Gln Glu Phe Thr Val Pro Gly Ser
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Gln Met

3. A recombinant plasmid which contains DNA coding for a polypeptide of Claim 1 or 2.

4. A transformant which carries a recombinant plasmid of Claim 3.

5. A method for the preparation of a polypeptide which comprises cultivating a transformant of Claim 4 and recovering a polypeptide of Claim 1 or 2 from the culture medium.

## Patentansprüche

1. Polypeptid mit Zellen-ausbreitender Aktivität, das eine Aminosäuresequenz der folgenden allgemeinen Formel hat: 
X-Glu Gln His Glu Ser Thr Pro Leu Arg Gly
Arg Gln Lys Thr Gly Leu Asp Ser Pro Thr
Gly Ile Asp Phe Ser Asp Ile Thr Ala Asn
Ser Phe Thr Val His Trp Ile Ala Pro Arg
Ala Thr Ile Thr Gly Tyr Arg Ile Arg His
His Pro Glu His Phe Ser Gly Arg Pro Arg
Glu Asp Arg Val Pro His Ser Arg Asn Ser
Ile Thr Leu Thr Asn Leu Thr Pro Gly Thr
Glu Tyr Val Val Ser Ile Val Ala Leu Asn
Gly Arg Glu Glu Ser Pro Leu Leu Ile Gly
Gln Gln Ser Thr Val Ser Asp Val Pro Arg
Asp Leu Glu Val Val Ala Ala Thr Pro Thr
Ser Leu Leu Ile Ser Trp Asp Ala Pro Ala
Val Thr Val Arg Tyr Tyr Arg Ile Thr Tyr
Gly Glu Thr Gly Gly Asn Ser Pro Val Gln
Glu Phe Thr Val Pro Gly Ser Lys Ser Thr
 
Ala Thr Ile Ser Gly Leu Lys Pro Gly Val
Asp Tyr Thr Ile Thr Val Tyr Ala Val Thr
Gly Arg Gly Asp Ser Pro Ala Ser Ser Lys
Pro Ile Ser Ile Asn Tyr Arg Thr Glu Ile
Asp Lys Pro Ser Gln Met-Y,
 worin X eine Sequenz der folgenden Formel (II) ist: 
Val Pro Pro Pro Thr Asp Leu Arg Phe Thr
Asn Ile Gly Pro Asp Thr Met Arg Val Thr
Trp Ala Pro Pro Pro Ser Ile Asp Leu Thr
Asn Phe Leu Val Arg Tyr Ser Pro Val Lys
Asn Glu Glu Asp Val Ala Glu Leu Ser Ile
Ser Pro Ser Asp Asn Ala Val Val Leu Thr
Asn Leu Leu Pro Gly Thr Glu Tyr Val Val
Ser Val Ser Ser Val Tyr
 oder diese Sequenz (II), bei der eine gewisse Aminosäure oder ein Peptid oder gewisse Aminosäuren oder Peptide vom N-Terminus deletiert sind und Y entweder Cystein oder die Deletion von Cystein bedeutet.

2. Polypeptid mit Zellen-ausbreitender Aktivität gemäß Anspruch 1, dadurch gekennzeichnet, daß es die folgende Aminosäuresequenz hat:

Pro Thr Asp Leu Arg Phe Thr Asn Ile Gly
Pro Asp Thr Met Arg Val Thr Trp Ala Pro
Pro Pro Ser Ile Asp Leu Thr Asn Phe Leu
Val Arg Tyr Ser Pro Val Lys Asn Glu Glu
Asp Val Ala Glu Leu Ser Ile Ser Pro Ser
Asp Asn Ala Val Val Leu Thr Asn Leu Leu
Pro Gly Thr Glu Tyr Val Val Ser Val Ser
Ser Val Tyr Glu Gln His Glu Ser Thr Pro
Leu Arg Gly Arg Gln Lys Thr Gly Leu Asp
Ser Pro Thr Gly Ile Asp Phe Ser Asp Ile
Thr Ala Asn Ser Phe Thr Val His Trp Ile
Ala Pro Arg Ala Thr Ile Thr Gly Tyr Arg
Ile Arg His His Pro Glu His Phe Ser Gly
Arg Pro Arg Glu Asp Arg Val Pro His Ser
Arg Asn Ser Ile Thr Leu Thr Asn Leu Thr
Pro Gly Thr Glu Tyr Val Val Ser Ile Val
Ala Leu Asn Gly Arg Glu Glu Ser Pro Leu
Leu Ile Gly Gln Gln Ser Thr Val Ser Asp
Val Pro Arg Asp Leu Glu Val Val Ala Ala
Thr Pro Thr Ser Leu Leu Ile Ser Trp Asp

Ala Pro Ala Val Thr Val Arg Tyr Tyr Arg
Ile Thr Tyr Gly Glu Thr Gly Gly Asn Ser
Pro Val Gln Glu Phe Thr Val Pro Gly Ser
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Gln Met

3. Rekombinantes Plasmid, das eine DNA enthält, die für ein Polypeptid nach Anspruch 1 oder 2 codiert.

4. Transformante, die ein rekombinantes Plasmid nach Anspruch 3 aufweist.

5. Verfahren zur Herstellung eines Polypeptids, dadurch gekennzeichnet, daß man eine Transformante nach Anspruch 4 kultiviert und ein Polypeptid nach Anspruch 1 oder 2 aus dem Kulturmedium gewinnt.

## Revendications

1. Polypeptide ayant une activité de propagation cellulaire, qui possède une séquence d'amino-acides représentée par la formule générale : 
X-Glu Gln His Glu Ser Thr Pro Leu Arg Gly
Arg Gln Lys Thr Gly Leu Asp Ser Pro Thr
Gly Ile Asp Phe Ser Asp Ile Thr Ala Asn
Ser Phe Thr Val His Trp Ile Ala Pro Arg
Ala Thr Ile Thr Gly Tyr Arg Ile Arg His
His Pro Glu His Phe Ser Gly Arg Pro Arg
Glu Asp Arg Val Pro His Ser Arg Asn Ser
Ile Thr Leu Thr Asn Leu Thr Pro Gly Thr
Glu Tyr Val Val Ser Ile Val Ala Leu Asn
Gly Arg Glu Glu Ser Pro Leu Leu Ile Gly
Gln Gln Ser Thr Val Ser Asp Val Pro Arg
Asp Leu Glu Val Val Ala Ala Thr Pro Thr
Ser Leu Leu Ile Ser Trp Asp Ala Pro Ala
Val Thr Val Arg Tyr Tyr Arg Ile Thr Tyr
Gly Glu Thr Gly Gly Asn Ser Pro Val Gln
Glu Phe Thr Val Pro Gly Ser Lys Ser Thr
Ala Thr Ile Ser Gly Leu Lys Pro Gly Val
Asp Tyr Thr Ile Thr Val Tyr Ala Val Thr
Gly Arg Gly Asp Ser Pro Ala Ser Ser Lys
Pro Ile Ser Ile Asn Tyr Arg Thr Glu Ile
Asp Lys Pro Ser Gln Met-Y,
 dans laquelle X représente une séquence répondant à la formule (II) suivante : 
Val Pro Pro Pro Thr Asp Leu Arg Phe Thr
Asn Ile Gly Pro Asp Thr Met Arg Val Thr
Trp Ala Pro Pro Pro Ser Ile Asp Leu Thr
Asn Phe Leu Val Arg Tyr Ser Pro Val Lys
Asn Glu Glu Asp Val Ala Glu Leu Ser Ile
Ser Pro Ser Asp Asn Ala Val Val Leu Thr
Asn Leu Leu Pro Gly Thr Glu Tyr Val Val
Ser Val Ser Ser Val Tyr
 ou bien ladite séquence (II) avec un ou plusieurs aminoacides ou peptides éliminés de l'extrémité N-terminale, et Y représente la cystéine ou sa délétion.

2. Polypeptide ayant une activité de propagation cellulaire suivant la revendication 1, qui possède la séquence d'amino-acides suivante :

Pro Thr Asp Leu Arg Phe Thr Asn Ile Gly
Pro Asp Thr Met Arg Val Thr Trp Ala Pro
Pro Pro Ser Ile Asp Leu Thr Asn Phe Leu
Val Arg Tyr Ser Pro Val Lys Asn Glu Glu
Asp Val Ala Glu Leu Ser Ile Ser Pro Ser
Asp Asn Ala Val Val Leu Thr Asn Leu Leu
Pro Gly Thr Glu Tyr Val Val Ser Val Ser
Ser Val Tyr Glu Gln His Glu Ser Thr Pro

Leu Arg Gly Arg Gln Lys Thr Gly Leu Asp
Ser Pro Thr Gly Ile Asp Phe Ser Asp Ile
Thr Ala Asn Ser Phe Thr Val His Trp Ile
Ala Pro Arg Ala Thr Ile Thr Gly Tyr Arg
Ile Arg His His Pro Glu His Phe Ser Gly
Arg Pro Arg Glu Asp Arg Val Pro His Ser
Arg Asn Ser Ile Thr Leu Thr Asn Leu Thr
Pro Gly Thr Glu Tyr Val Val Ser Ile Val
Ala Leu Asn Gly Arg Glu Glu Ser Pro Leu
Leu Ile Gly Gln Gln Ser Thr Val Ser Asp
Val Pro Arg Asp Leu Glu Val Val Ala Ala
Thr Pro Thr Ser Leu Leu Ile Ser Trp Asp
Ala Pro Ala Val Thr Val Arg Tyr Tyr Arg
Ile Thr Tyr Gly Glu Thr Gly Gly Asn Ser
Pro Val Gln Glu Phe Thr Val Pro Gly Ser
Lys Ser Thr Ala Thr Ile Ser Gly Leu Lys
Pro Gly Val Asp Tyr Thr Ile Thr Val Tyr
Ala Val Thr Gly Arg Gly Asp Ser Pro Ala
Ser Ser Lys Pro Ile Ser Ile Asn Tyr Arg
Thr Glu Ile Asp Lys Pro Ser Gln Met

3. Plasmide recombinant qui contient un ADN codant pour un polypeptide suivant la revendication 1 ou 2.

4. Transformant qui porte un plasmide recombinant suivant la revendication 3.

5. Procédé de préparation d'un polypeptide, qui comprend la culture d'un transformant suivant la revendication 4 et la séparation d'un polypeptide suivant la revendication 1 ou 2 du milieu de culture.
